(19) Europäisches Patentamt
European Patent Office
Office européen des brevets

(11) EP 1 071 394 B1

(12) **EUROPÄISCHE PATENTSCHRIFT**

(45) Veröffentlichungstag und Bekanntmachung des Hinweises auf die Patenterteilung:
**14.07.2004 Patentblatt 2004/29**

(21) Anmeldenummer: **99920689.9**

(22) Anmeldetag: **16.04.1999**

(51) Int Cl.[7]: **A61K 7/42**

(86) Internationale Anmeldenummer:
**PCT/EP1999/002581**

(87) Internationale Veröffentlichungsnummer:
**WO 1999/053895 (28.10.1999 Gazette 1999/43)**

(54) **VERWENDUNG UNSYMMETRISCH SUBSTITUIERTEN TRIAZINDERIVATEN IN KOSMETISCHE ODER DERMATOLOGISCHE ZUBEREITUNGEN ZUM ERHALT DES UROCANINSÄURESTATUS DER HAUT**

USE OF UNSYMMETRICALLY SUBSTITUTED TRIAZINE DERIVATIVES IN COSMETIC OR DERMATOLOGICAL PREPARATIONS FOR MAINTAINING THE UROCANIC ACID STATUS OF THE SKIN

Utilisation de dérivés de triazine substitués de façon asymetrique dans des préparations cosmetiques ou dermatologiques en vue de conserver l’acide urocanique sur la peau

(84) Benannte Vertragsstaaten:
**DE ES FR GB IT SE**

(30) Priorität: **18.04.1998 DE 19817295**

(43) Veröffentlichungstag der Anmeldung:
**31.01.2001 Patentblatt 2001/05**

(73) Patentinhaber: **Beiersdorf Aktiengesellschaft**
**20245 Hamburg (DE)**

(72) Erfinder:
- **GERS-BARLAG, Heinrich**
  **D-25495 Kummerfeld (DE)**
- **HARGENS, Birgit**
  **D-20257 Hamburg (DE)**
- **MÜLLER, Anja**
  **D-23843 Rümpel (DE)**

(56) Entgegenhaltungen:
**EP-A- 0 570 838**
**EP-A- 0 850 935**
**WO-A-99/06014**
**DE-A- 4 230 076**
**DE-A- 4 307 983**
**DE-A- 4 405 585**
**DE-A- 4 429 468**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 071 394 B1

Printed by Jouve, 75001 PARIS (FR)

## Beschreibung

Die vorliegende Erfindung betrifft kosmetische und dermatologische Zubereitungen mit einem Gehalt an unsymmetrisch substituierten Triazinderivaten. In weiteren Ausführungsformen betrifft die vorliegende Erfindung Zubereitungen zum Schutze der Haut gegen UV-Strahlung. Ferner betrifft die Erfindung dermatologische Zubereitungen, die den Immunstatus der menschlichen Haut erhalten und verbessern.

Unter Hautpflege ist in erster Linie zu verstehen, daß die natürliche Funktion der Haut als Barriere gegen Umwelteinflüsse (z.B. Schmutz, Chemikalien, Mikroorganismen) und gegen den Verlust von körpereigenen Stoffen (z. B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliches Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Zeichen der Hautalterung verzögern.

cis-Urocaninsäure (auch cis-Urocansaure oder cis-4-Imidazolylacrylsäure genannt) ist durch folgende Strukturformel gekennzeichnet:

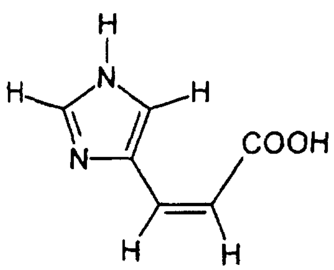

Sie hat die Summenformel $C_6H_6N_2O_2$ und die Molekularmasse 138,12. cis-Urocaninsäure entsteht beispielsweise durch UV-Bestrahlung des trans-Isomeren, welches in der menschlichen Haut und auch im Schweiß vorkommt.

Die trans-Urocaninsäure ist durch folgende Strukturformel gekennzeichnet:

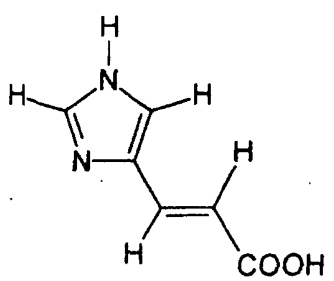

Die trans-Urocaninsäure hat ebenfalls die Summenformel $C_6H_6N_2O_2$ und die Molekularmasse 138,12 und kommt in der menschlichen Haut und auch im Schweiß vor.

Wenn im Rahmen der hiermit vorgelegten Offenbarung der Begriff "Urocaninsäure" ohne einen Hinweis auf das betreffende Isomere angewandt wird, so werden davon sowohl das cis- als auch das trans-Isomere sowie beliebige Mischungen beider Isomeren erfaßt.

Die DE-OS 41 21 030 zeigt, daß Urocaninsäure antiphlogistisch wirkt, die Folgen allergischer Reaktionen mildert und in hohem Maße allergischen Reaktionen vorbeugt.

Aufgrund antiphlogistischer und antiallergischer Potenz ist die Urocaninsäure wirksam gegen Psoriasis, Neurodermitis und Kontaktdermatitis und Autoimmunkrankheiten, wie z.B. Vitiligo, Pruritus, Alopecia areata, Ichthyose sowie Atopie, bei denen ein ähnlicher Wirkmechanismus vorliegt.

Die herkömmlichen Zubereitungen konnten nicht verhindern, daß die hauteigene Urocaninsäure beim Kontakt mit Wasser und/oder Tensiden oder beim Schwitzen aus- oder abgewaschen wird. Auch herkömmliche Zubereitungen mit einem Gehalt an Urocaninsäure (siehe z.B. DE-OS-4230076, DE-OS-4307983, DE-OS-4405585) konnten den Urocaninsäurestatus der Haut bestenfalls ein wenig auffrischen, den ursprünglichen Zustand selbst aber nicht mehr

erreichen. Dieser war bisher nur nach der individuellen Regenerationszeit des Betroffenen erreichbar.

Zubereitungen zur Pflege der Haut liegen meist in Form von Crèmes, Lotionen, Milchen, Salben, Salbengrundlagen, Ölen, Tinkturen, Stiften, Spray-Formulierungen und dergleichen vor.

Gebräuchliche kosmetische Zubereitungen beispielsweise sind Sonnenschutzmittel. Die Verwendung der trans-Urocaninsäure als Sonnenschutzmittel ist ebenfalls bekannt.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen der Haut.

Als ein Maximum der Erythemwirksamkeit des Sonnenlichtes wird der engere Bereich um 308 nm angesehen.

Zum Schutze gegen UVB-Strahlung sind zahlreiche Verbindungen bekannt, bei denen es sich zumeist um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Wellenlängenbereich zwischen etwa 320 und 400 nm, den sogenannten UVA-Bereich, sind UV-Filtersubstanzen wichtig, da auch solche Strahlen Schäden hervorrufen können. So ist erwiesen, daß UVA-Strahlung zu einer Schädigung der elastischen und kollagenen Fasern des Bindegewebes führt, was die Haut vorzeitig altern läßt und als Ursache zahlreicher phototoxischer und photoallergischer Reaktionen zu sehen ist. Der schädigende Einfluß der UVB-Strahlung kann durch UVA-Strahlung verstärkt werden.

Die UV-Strahlung kann aber auch zu photochemischen Reaktionen führen, wobei dann die photochemischen Reaktionsprodukte in den Haut- und Zellmetabolismus eingreifen.

Vorwiegend handelt es sich bei solchen photochemischen Reaktionsprodukten um radikalische Verbindungen, z.B. Hydroxyradikale, Hydroperoxyradikale sowie Superoxidionen. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikatischer angeregter Zustand des Sauerstoffmoleküls, kann bei UV-Bestrahlung auftreten, ebenso kurzlebige Epoxide und viele Andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Um diesen Reaktionen vorzubeugen, können den kosmetischen bzw. dermatologischen Formulierungen zusätzlich Antioxidantien und/oder Radikalfänger einverleibt werden.

UV-Absorber bzw. UV-Reflektoren sind die meisten anorganischen Pigmente, die bekannterweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen.

Obwohl es durchaus vorteilhafte kosmetische bzw. dermatologische Zubereitungen zum Schutze der Haut vor den schädlichen Folgen der Einwirkung von UV-Licht gibt, ist ein oft beobachteter Nachteil, daß die Zubereitungen nicht oder nicht hinreichend wasserfest sind.

Lichtschutzzubereitungen werden besonders häufig an Badestränden bzw. in Freibädern benötigt und angewandt. Wünschenswert ist dann, daß die Lichtschutzformulierung weitgehend wasserfest ist, daß sie also nicht oder nur in geringem Maße von der Haut abgewaschen wird.

Höhere Lichtschutzfaktoren, also etwa solche die oberhalb von LF 15 angesiedelt sind, lassen sich im allgemeinen nur durch hohe Mengen an UV-Filtersubstanzen erreichen. Soll ein Sonnenschutzprodukt auch nach dem Baden noch einen hohen Lichtschutzfaktor aufweisen, muß insbesondere die UV-Filtersubstanz auf der Haut erhalten bleiben.

Es ist an sich schon lästig, wenn nach dem Baden das Sonnenschutzprodukt erneut aufgetragen werden muß. Beim Baden selbst kann die Verwendung einer abwaschbaren Lichtschutzformulierung unter Umständen sogar leichtsinnig und schädlich für die Haut sein, da Wasser das Licht im UVA- und UVB-Bereich schlecht absorbiert, infolgedessen keinen nennenswerten UV-Schutz darstellt, nicht einmal für untergetauchte Hautbereiche.

Für wasserfeste Lichtschutzformulierungen verwendet der Stand der Technik üblicherweise nichtwasserlösliche UV-Filtersubstanzen, wasserabweisende Rohstoffe (z.B. Siliconöle in hohen Konzentrationen) und/oder Filmbildner, insbesondere hochmolekulare Verbindungen (z.B. PVP/Hexadecen-Copolymere). Dabei werden Barrieren zwischen den auf der Haut aufliegenden UV-Filtersubstanzen und dem Wasser aufgebaut.

Nachteilig dabei ist, daß die Diffusion der Filtersubstanzen ins Wasser zwar verzögert, aber nicht vollständig verhindert werden kann. Deshalb können derartige Produkte bei längerem Baden beachtlich an Schutzwirkung verlieren. Aber selbst durch normale Schweißentwicklung bzw. Abwischen dieses Schweißes und der darin gelösten bzw. angelösten Lichtschutzsubstanzen, insbesondere der hauteigenen, aber auch der künstlich aufgetragenen Urocaninsäure, kann erhebliche Verminderung des Lichtschutzes eintreten.

Schon bei einem einfachen Wasserbade ohne Zusatz von Tensiden kommt es zunächst zu einer Quellung der Hornschicht der Haut, wobei der Grad dieser Quellung beispielsweise von der Dauer des Bades und dessen Temperatur abhängt. Zugleich werden wasserlösliche Stoffe, z.B. wasserlösliche Schmutzbestandteile, aber auch hauteigene Stoffe, die für das Wasserbindungsvermögen der Hornschicht verantwortlich sind, ab- bzw. ausgewaschen. Durch hauteigene tensioaktive Stoffe bewirkt, werden zudem auch Hautfette in gewissem Ausmaße gelöst und ausgewaschen. Dies bedingt nach anfänglicher Quellung eine nachfolgende deutliche Austrocknung der Haut, die durch waschaktive Zusätze nach verstärkt werden kann. Insbesondere die hauteigene Urocaninsäure kann aufgrund ihrer hohen Hydrophilie leicht aus der Haut ausgewaschen werden.

Bei gesunder Haut sind diese Vorgänge im allgemeinen belanglos, da die Schutzmechanismen der Haut solche leichten Störungen der oberen Hautschichten ohne weiteres kompensieren können. Aber bereits im Falle nichtpathologischer Abweichungen vom Normalstatus, z.B. durch umweltbedingte Abnutzungsschäden bzw. Irritationen, Lichtschäden, Altershaut usw., ist der Schutzmechanismus der Hautoberfläche gestört. Unter Umständen ist er dann aus eigener Kraft nicht mehr imstande, seine Aufgabe zu erfüllen und muß durch externe Maßnahmen regeneriert werden.

Zwar beschreibt die DE-OS 44 29 468 kosmetische und dermatologische Zubereitungen mit einem Gehalt an hydrophobierten anorganischen Pigmenten zum Erhalt des Urocaninsäurestatus der Haut, doch sind auch die dort beschriebenen Zubereitungen verbesserungsfähig.

Von verschiedenen Autoren wurden UV-Filtersubstanzen vorgestellt, welche das Strukturmotiv

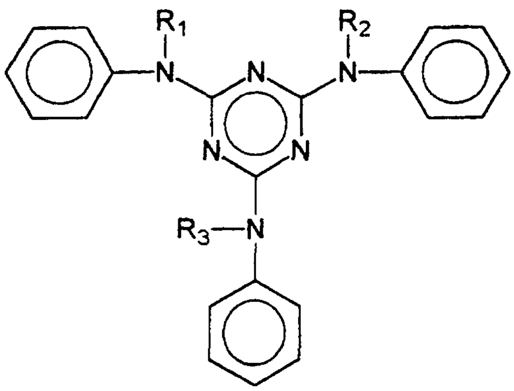

aufweisen.

Hinsichtlich der $C_3$-Achse des Triazingrundkörpers sind sowohl symmetrische Substitution wie auch unsymmetrische Substitution denkbar. In diesem Sinne symmetrisch substituierte s-Triazine weisen drei gleiche Substituenten auf und werden beispielsweise vertreten durch den 4,4',4"-(1,3,5-Triazin-2,4,6-triyltriimino)-tris-benzoesäure-tris (2-ethylhexylester), analog der INCI-Nomenklatur auch: Octyltriazon, welcher durch folgende Struktur wiedergegeben wird:

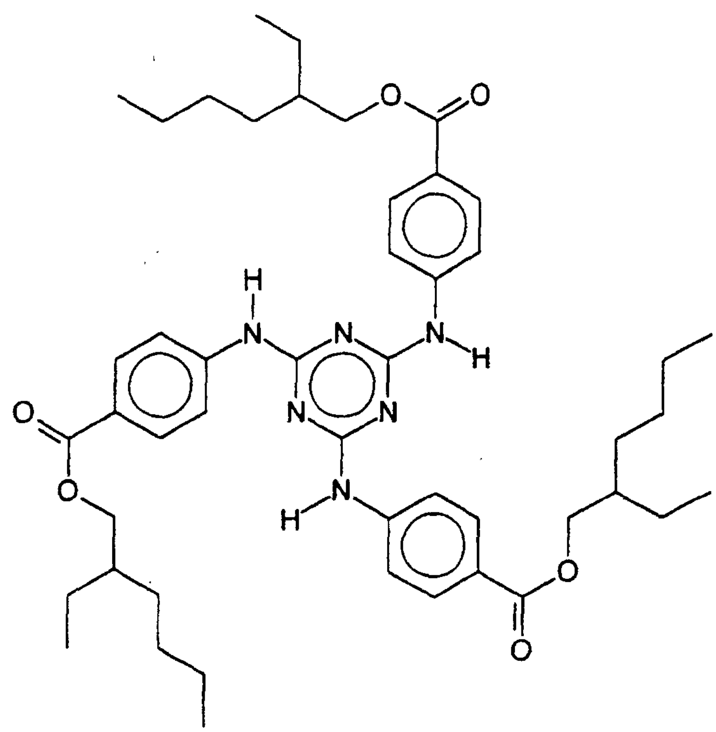

Hinsichtlich der $C_3$-Achse unsymmetrisch substituierte s-Triazinderivate weisen demzufolge unterschiedliche Substituenten auf, wodurch die $C_3$-Symmetrie zerstört wird. Im Sinne der hiermit vorliegenden Erfindung wird als "symmetrisch" bzw. "unsymmetrisch" stets symmetrisch bzw. unsymmetrisch hinsichtlich der $C_3$-Achse des Triazingrundkörpers verstanden, es sei denn, etwas Anderes wäre ausdrücklich erwähnt.

So werden in der EP-A- 570 838 unsymmetrisch substituierte s-Triazinderivate beschrieben, deren chemische Struktur durch die generische Formel

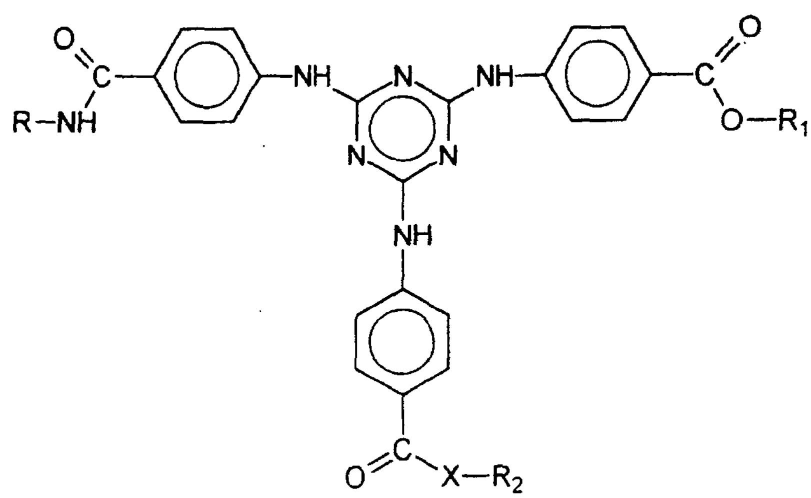

wiedergegeben wird, wobei

- R einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, darstellt,
- X ein Sauerstoffatom oder eine NH-Gruppe darstellt,
- $R_1$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, oder ein Wasserstoffatom, ein Alkalimetallatom, eine Ammoniumgruppe oder eine Gruppe der Formel

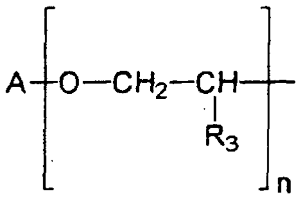

bedeutet, in welcher

- A einen verzweigten oder unverzweigten $C_1$-$C_{18}$-Alkylrest, einen $C_5$-$C_{12}$-Cycloalkyl- oder Arylrest darstellt, gegebenenfalls substituiert mit einer oder mehreren $C_1$-$C_4$- Alkylgruppen,
- $R_3$ ein Wasserstoffatom oder eine Methylgruppe darstellt,
- n eine Zahl von 1 bis 10 darstellt,

$R_2$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, darstellt, wenn X die NH-Gruppe darstellt, und einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, oder ein Wasserstoffatom, ein Alkalimetallatom, eine Ammoniumgruppe oder eine Gruppe der Formel

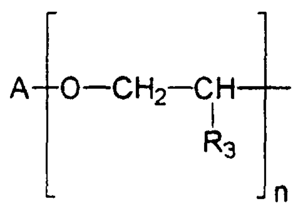

bedeutet, in weicher

- A einen verzweigten oder unverzweigten $C_1$-$C_{18}$-Alkylrest, einen $C_5$-$C_{12}$-Cycloalkyl- oder Arylrest darstellt, gegebenenfalls substituiert mit einer oder mehreren $C_1$-$C_4$- Alkylgruppen,
- $R_3$ ein Wasserstoffatom oder eine Methylgruppe darstellt,
- n eine Zahl von 1 bis 10 darstellt,

wenn X ein Sauerstoffatom darstellt.

Solche s-Triazinderivate, die sich im Gegensatze zum 4,4',4"-(1,3,5-Triazin-2,4,6-triyltriimino)-tris-benzoesäure-tris(2-ethylhexylester) durch verbesserte Löslichkeit in vielen Ölkomponenten auszeichnen, können gemäß der Lehre der Schriften EP-A-821 937, EP-A-821 938, EP-A-821 939, EP-A-821 940 und EP-A-821 941 mit verschiedenen anderen Lichtschutzfiltern in kosmetischen oder dermatologischen Zubereitungen kombiniert werden, wodurch bestimmten technischen Sachverhalten Rechnung getragen werden soll.

Eine Aufgabe der vorliegenden Erfindung war es also, all diesen Übelständen Abhilfe zu schaffen. Aufgabe der Erfindung war es insbesondere, Zubereitungen zur Verfügung zu stellen, welche, gleichgültig, ob ihnen ein zusätzlicher Gehalt an Urocaninsäure beigemischt wird oder nicht, gewährleisten, daß nach Kontakt mit Wasser der Urocaninsäurestatus der Haut möglichst wenig beeinträchtigt, oder, im Falle eines akuten Mangels an Urocaninsäure, ein nahezu physiologischer Urocaninsäurestatus erreicht wird. Weiterhin wer eine Aufgabe der vorliegenden Erfindung, Lichtschutzzubereitungen zur Verfügung zu stellen, die sich durch eine hohe Lichtschutzwirkung und gute kosmetische und dermatologische Akzeptanz auszeichnen.

Erstaunlicherweise werden alle diese Aufgaben gelöst durch die Verwendung von einem oder mehreren unsymmetrisch substituierten s-Triazinderivaten in kosmetischen oder dermatologischen Zubereitungen zur Verhinderung

- des durch Einwirkung von Wasser hervorgerufenen Aus- oder Abwaschens der hauteigenen cis- bzw. trans-Urocaninsäure von oder aus der menschlichen Haut oder
- des durch Einwirkung von Wasser hervorgerufenen Aus- oder Abwaschens von künstlich auf die Haut aufgetragener cis- bzw. trans-Urocaninsäure von oder aus der menschlichen Haut.

Erfindungsgemäß ist vorteilhaft, urocaninsäurefreie Zubereitungen zu wählen. Es kann aber gegebenenfalls auch vorteilhaft sein, in erfindungsgemäße Zubereitungen einen Gehalt an cis- und/oder trans-Urocaninsäure einzusetzen.

In einer vorteilhaften Ausführungsform betrifft die vorliegende Erfindung die Verwendung von einem oder mehreren unsymmetrisch substituierten s-Triazinderivaten in kosmetischen oder dermatologischen Zubereitungen zur Verhinderung

- des durch Einwirkung von Wasser hervorgerufenen Aus- oder Abwaschens der hauteigenen cis- bzw. trans-Urocaninsäure von oder aus der menschlichen Haut oder
- des durch Einwirkung von Wasser hervorgerufenen Aus- oder Abwaschens von künstlich auf die Haut aufgetragener cis- bzw. trans-Urocaninsäure von oder aus der menschlichen Haut,

das dadurch gekennzeichnet ist, daß das oder die unsymmetrisch substituierten s-Triazinderivate gewählt werden aus der Gruppe der Substanzen, deren chemische Struktur durch die generische Formel

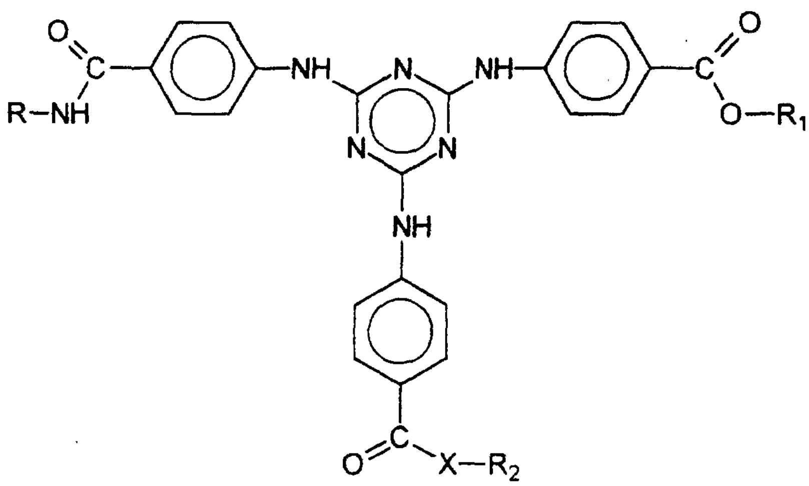

wiedergegeben wird, wobei

R einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, darstellt,

X ein Sauerstoffatom oder eine NH-Gruppe darstellt,

$R_1$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, oder ein Wasserstoffatom, ein Alkalimetallatom, eine Ammoniumgruppe oder eine Gruppe der Formel

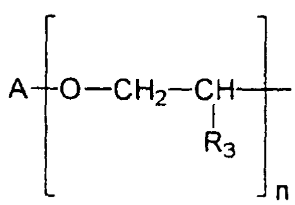

bedeutet, in welcher

A einen verzweigten oder unverzweigten $C_1$-$C_{18}$-Alkylrest, einen $C_5$-$C_{12}$-Cycloalkyl- oder Arylrest darstellt, gegebenenfalls substituiert mit einer oder mehreren $C_1$-$C_4$- Alkylgruppen,

$R_3$ ein Wasserstoffatom oder eine Methylgruppe darstellt,

n eine Zahl von 1 bis 10 darstellt,

$R_2$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, darstellt, wenn X die NH-Gruppe darstellt, und einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, oder ein Wasserstoffatom, ein Alkalimetallatom, eine Ammoniumgruppe oder eine Gruppe der Formel

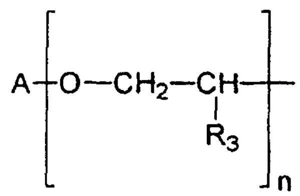

bedeutet, in welcher

- A einen verzweigten oder unverzweigten $C_1$-$C_{18}$-Alkylrest, einen $C_5$-$C_{12}$-Cycloalkyl- oder Arylrest darstellt, gegebenenfalls substituiert mit einer oder mehreren $C_1$-$C_4$- Alkylgruppen,
- $R_3$ ein Wasserstoffatom oder eine Methylgruppe darstellt,
- n eine Zahl von 1 bis 10 darstellt,

wenn X ein Sauerstoffatom darstellt.

In einer bevorzugten Ausführungsform betrifft die vorliegende Erfindung die Verwendung des Dioctylbutylamidotriazons, dessen chemische Struktur durch die Formel

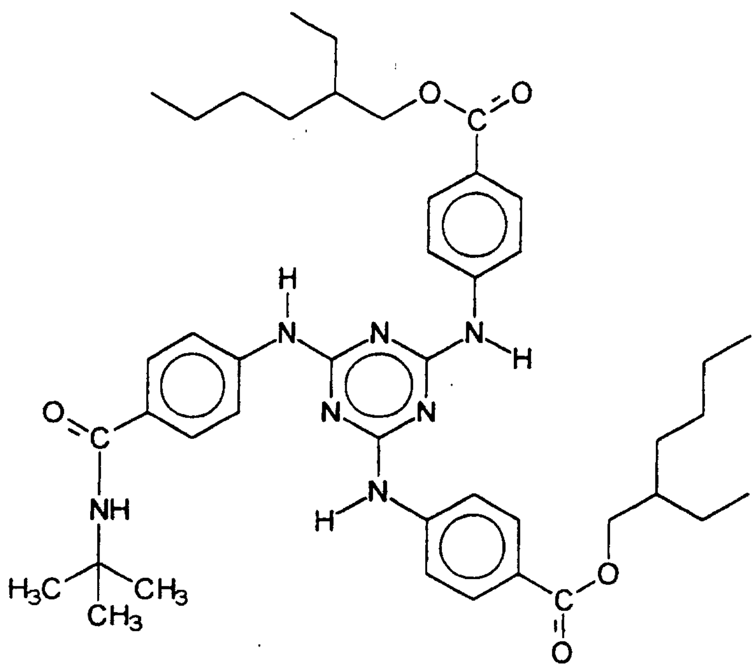

wiedergegeben wird, zur Verhinderung

- des durch Einwirkung von Wasser hervorgerufenen Aus- oder Abwaschens der hauteigenen cis- bzw. trans-Urocaninsäure von oder aus der menschlichen Haut oder
- des durch Einwirkung von Wasser hervorgerufenen Aus- oder Abwaschens von künstlich auf die Haut aufgetragener cis- bzw. trans-Urocaninsäure von oder aus der menschlichen Haut.

Medizinische topische Zusammensetzungen enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Es war überraschend und für den Fachmann nicht zu erwarten, daß gerade die als lipophil anzusehenden s-Triazinderivate die erfindungsgemäß vorteilhaften Eigenschaften aufweisen, da die Urocaninsäure andererseits durchaus als hydrophile Substanz zu bezeichnen ist

Schließlich ist es überraschend, daß es für praktische Zwecke unerheblich ist, ob die erfindungsgemäßen Zubereitungen in bezug auf die Formulierung selbst als "wasserfest" (also beispielsweise unter Verwendung eines ausgeprägten Gehaltes an wasserunlöslichen Filmbildnern) oder als "nicht wasserfest" (also beispielsweise ohne einen solchen Gehalt) zu gelten haben. Der Urocaninsäurestatus der Haut ist erfindungsgemäß ohne Verwendung von filmbildenden Zubereitungen und nach provokativer Wasseranwendung kaum schlechter als bei solcher Verwendung von filmbildenden Zubereitungen.

Es ist daher erfindungsgemäß möglich und gegebenenfalls besonders vorteilhaft, auf einen Gehalt an Filmbildner zu verzichten, und zwar sowohl auf lipophile wie auch auf hydrophile Filmbildner.

Die Herstellung erfindungsgemäßer Zubereitungen geschieht nach den üblichen, dem Fachmanne geläufigen Regeln. Vorteilhaft liegen die erfindungsgemäßen Zubereitungen als Emulsionen, vorteilhaft W/O-Emulsionen, bevorzugt O/W-Emulsionen, vor. Es ist aber auch möglich und erfindungsgemäß gegebenenfalls vorteilhaft, andere Formulierungsarten zu wählen, beispielsweise Hydrodispersionen, Gele, Öle, Multiple Emulsionen, beispielsweise in Form von W/O/W- oder O/W/O-Emulsionen, wasserfreie Salben bzw. Salbengrundlagen usw.

In einfachen Emulsionen liegen in der einen Phase feindisperse, von einer Emulgatorhülle umschlossene Tröpfchen der zweiten Phase (Wassertröpfchen in W/O- oder Lipidvesikel in O/W-Emulsionen) vor. In einer multiplen Emulsion (zweiten Grades) hingegen sind in solchen Tröpfchen feiner disperse Tröpfchen der ersten Phase emulgiert. Auch in diesen Tröpfchen wiederum können noch feiner disperse Tröpfchen vorliegen (multiple Emulsion dritten Grades) und so fort.

So wie man also bei den einfachen Emulsionen von W/O- oder O/W-Emulsionen spricht (Wasser-in-Oel oder Oel-in-Wasser), gibt es bei multiplen Emulsionen W/O/W-, O/W/O-, O/W/O/W-, W/O/W/O-Emulsionen und so fort.

Hydrodispersionen stellen Dispersionen einer flüssigen, halbfesten oder festen inneren (diskontinuierlichen) Lipidphase in einer äußeren wäßrigen (kontinuierlichen) Phase dar.

Im Gegensatze zu O/W-Emulsionen, die sich durch eine ähnliche Phasenanordnung auszeichnen, sind Hydrodispersionen aber im wesentlichen frei von Emulgatoren. Hydrodispersionen stellen, wie im übrigen auch Emulsionen metastabile Systeme dar, und sind geneigt, in einen Zustand zweier in sich zusammenhängender diskreter Phasen überzugehen. In Emulsionen verhindert die Wahl eines geeigneten Emulgators die Phasentrennung.

Bei Hydrodispersionen einer flüssigen Lipidphase in einer äußeren wäßrigen Phase kann die die Stabilität eines solchen Systems beispielsweise dadurch gewährleistet werden, daß in der wäßrigen Phase ein Gelgerüst aufgebaut wird, in welchem die Lipidtröpfchen stabil suspendiert sind.

Die Gesamtmenge an einem oder mehreren unsymmetrisch substituierten s-Triazinderivaten, insbesondere von Dioctylbutylamidotriazon, in den fertigen kosmetischen oder dermatologischen Zubereitungen wird vorteilhaft aus dem Bereich von 0,1 - 15,0 Gew.-%, bevorzugt 0,5 - 8,0 Gew.-% gewählt, bezogen auf das Gesamtgewicht der Zubereitungen.

Erfindungsgemäße kosmetische und dermatologische Zubereitungen enthalten vorteilhaft außerdem anorganische Pigmente auf Basis von Metalloxiden und/oder anderen in Wasser schwerlöslichen oder unlöslichen Metallverbindungen, insbesondere der Oxide des Titans ($TiO_2$), Zinks (ZnO), Eisens (z.B. $Fe_2O_3$), Zirkoniums ($ZrO_2$), Siliciums ($SiO_2$), Mangans (z.B. MnO), Aluminiums ($Al_2O_3$), Cers (z.B. $Ce_2O_3$), Mischoxiden der entsprechenden Metalle sowie Abmischungen aus solchen Oxiden. Besonders bevorzugt handelt es sich um Pigmente auf der Basis von $TiO_2$.

Es ist besonders vorteilhaft im Sinne der vorliegenden Erfindung, wenngleich nicht zwingend, wenn die anorganischen Pigmente in hydrophober Form vorliegen, d.h., daß sie oberflächlich wasserabweisend behandelt sind. Diese Oberflächenbehandlung kann darin bestehen, daß die Pigmente nach an sich bekannten Verfahren mit einer dünnen hydrophoben Schicht versehen werden.

Eines solcher Verfahren besteht beispielsweise darin, daß die hydrophobe Oberflächenschicht nach einer Rektion gemäß

$$n\ TiO_2 + m\ (RO)_3\ Si\text{-}R' \text{ -> } n\ TiO_2\ (\text{oberfl.})$$

erzeugt wird. n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Vorteilhafte $TiO_2$-Pigmente sind beispielsweise unter den Handelsbezeichnungen MT 100 T von der Firma TAYCA, ferner M 160 von der Firma Kemira sowie T 805 von der Firma Degussa erhältlich.

Die erfindungsgemäßen kosmetischen und/oder dermatologischen Lichtschutzformulierungen können wie

üblich zusammengesetzt sein und dem kosmetischen und/oder dermatologischen Lichtschutz, ferner zur Behandlung, der Pflege und der Reinigung der Haut und/oder der Haare und als Schminkprodukt in der dekorativen Kosmetik dienen.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Besonders bevorzugt sind solche kosmetischen und dermatologischen Zubereitungen, die in der Form eines Sonnenschutzmittels vorliegen. Vorteilhaft können diese zusätzlich mindestens einen weiteren UVA-Filter und/oder mindestens einen weiteren UVB-Filter und/oder mindestens ein anorganisches Pigment, bevorzugt ein anorganisches Mikropigment, enthalten.

Die kosmetischen und dermatologischen Zubereitungen gemäß der Erfindung können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel. anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D, L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin. Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsutfone, Penta-, Hexa-, Heptathioninsulfoximin) in sehr geringen verträglichen Dosierungen (z.B. pmol bis μmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew .-%. insbesondere 1 - 10 Gew .-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Die Lipidphase kann vorteilhaft gewählt werden aus folgender Substanzgruppe:

- Mineralöle, Mineralwachse
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decytoleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12-15}$-Alkylbenzoat, Capryl-Caprinsäure-triglycerid, Dicaprylylether.

Besonders vorteilhaft sind Mischungen aus $C_{12-15}$-Alkybenzoat und 2-Ethylhexylisostearat, Mischungen aus $C_{12-15}$-Alkybenzoat und Isotridecylisononanoat sowie Mischungen aus $C_{12-15}$-Alkybenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonol eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wäßrige Phase der erfindungsgemäßen Zubereitungen enthält gegebenenfalls vorteilhaft

- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Ganz besonders vorteilhaft enthalten Zubereitungen gemäß der vorliegenden Erfindung eine oder mehrere grenzflächenaktive Substanzen A, gewählt aus der Gruppe der Glucosederivate, welche sich durch die Strukturformel

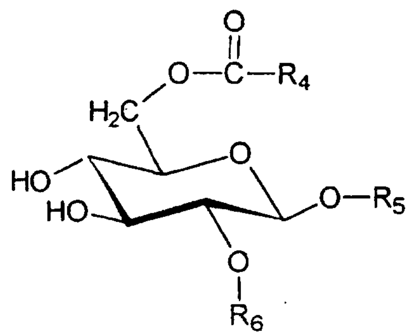

auszeichnen, wobei $R_4$ einen verzweigten oder unverzweigten Alkylrest mit 1 bis 24 Kohlenstoffatomen darstellt, wobei $R_5$ entweder ein Wasserstoffatom oder einen verzweigten oder unverzweigten Alkylrest mit 1 bis 24 Kohlenstoffatomen darstellt und wobei $R_6$ entweder ein Wasserstoffatom oder einen verzweigten oder unverzweigten Acylrest mit 1 bis 24 Kohlenstoffatomen darstellt, sowie.

gewünschtenfalls eine oder mehrere grenzflächenaktive Substanzen B, gewählt aus der Gruppe der Substanzen der allgemeinen Strukturformel

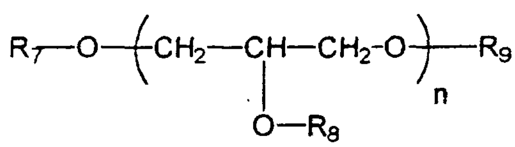

wobei $R_7$, $R_8$ und $R_9$ voneinander gewählt werden aus der Gruppe, welche umfaßt: H, verzweigte bzw. unverzweigte, gesättigte bzw. ungesättigte Fettsäurereste mit 8 bis 24 Kohlenstoffatomen, bei welchen bis zu drei aliphatische Wasserstoffatome durch Hydroxygruppen substituiert sein können und n eine Zahl von 2 bis 8 darstellt.

Vorteilhaft wird $R_4$ gewählt aus der Gruppe der unverzweigten Alkylreste, wobei der Myristylrest, der Palmitylrest, der Stearylrest und der Eicosylrest bevorzugt werden.

$R_5$ kann vorteilhaft ein Wasserstoffatom darstellen, wird aber bevorzugt aus der Gruppe Methyl-, Ethyl-, Propyl- und Isopropyl- gewählt.

$R_6$ kann vorteilhaft ein Wasserstoffatom darstellen, kann aber ebenfalls vorteilhaft aus der Gruppe Myristoyl-, Palmitoyl-, Stearoyl- und Eicosoyl- gewählt werden.

Besonders vorteilhaft wird oder werden die grenzflächenaktiven Substanzen A aus der Gruppe Methylglucosemonostearat (Formel wie nachstehend)

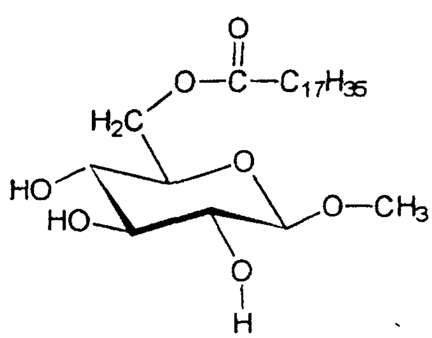

(A1)

Methylglucosedistearat (Formel wie nachstehend)

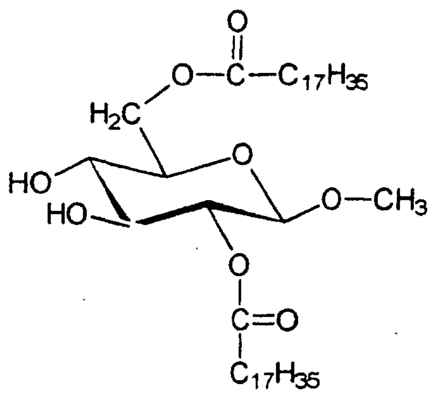

(A2).

und beliebigen Gemischen daraus, beispielsweise ungefähr äquimolaren Mischungen daraus gewählt, welche auch als Methylglucosesesquistearat bezeichnet werden. Solches Methylglucosesesquistearat ist im Handel beispielsweise unter der Warenbezeichnung Tego® Care PS von der. Gesellschaft Th.Goldschmidt KG erhältlich.

Ebenfalls besonders vorteilhaft wird oder werden die grenzflächenaktiven Substanzen B gewählt aus der Gruppe der Verbindungen, bei welcher n den Wert 3 annimmt und $R_3$, $R_4$ und $R_5$ unabhängig voneinander gewählt werden aus der Gruppe, welche umfaßt: H, verzweigte bzw. unverzweigte, gesättigte bzw. ungesättigte Fettsäurereste mit 14 bis 20 Kohlenstoffatomen, insbesondere die nachfolgend aufgeführten Strukturen:

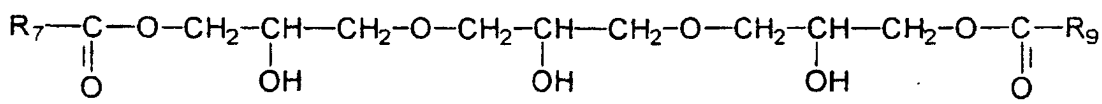

(B1).

Als erfindungsgemäß bevorzugte Emulgatorkombination hat sich ein ungefähr äquimolekulares Gemisch aus den Verbindungen A2 und B1, wobei in B1 die Reste $R_7$ und $R_9$ vorzugsweise beide einen Stearatrest bezeichnen, herausgestellt. Solche Emulgatorkombinationen sind als "Polyglyceryl(3)-Methylglucosedistearat" (PGMS) unter der Warenbezeichnung Tego Care® 450 von der Gesellschaft Th. Goldschmidt KG erhältlich.

Erfindungsgemäß können diese grenzflächenaktiven Substanzen A und/oder Bin Konzentrationen von 0,005 bis 50 Gew.%, bezogen auf das Gesamtgewicht der Zubereitungen. vorliegen. Dabei werden Konzentrationen von 0,5 - 10 Gew.-%, insbesondere 1,0 - 5 Gew.-%, bevorzugt.

Die kosmetischen oder dermatologischen Lichtschutzzubereitungen enthalten vorteilhaft anorganische Pigmente, insbesondere Mikropigmente, z.B. in Mengen von 0,1 Gew.-% bis 30 Gew.-%, vorzugsweise in Mengen von 0,5 Gew.-% bis 10 Gew.-%, insbesondere aber 1 Gew.-% bis 6 Gew.-%, bezogen auf das Gesamtgewicht der Zubereitungen.

Es ist erfindungsgemäß vorteilhaft, außer den erfindungsgemäßen Kombinationen öllösliche UVA-Filter und/oder UVB-Filter in der Lipidphase und/oder wasserlösliche UVA-Filter und/oder UVB-Filter in der wäßrigen Phase einzusetzen.

Vorteilhaft können die erfindungsgemäßen Lichtschutzformulierungen weitere Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, um kosmetische Zubereitungen zur Verfügung zu stellen, die die Haut vor dem gesamten Bereich der ultravioletten Strahlung schützen. Sie können auch als Sonnenschutzmittel dienen.

Die weiteren UVB-Filter können öllöslich oder wasserlöslich sein. Vorteilhafte öllösliche UVB-Filtersubstanzen sind z.B.:

- 3-Benzylidencampher-Derivate, vorzugsweise 3-(4-Methylbenzyliden)campher. 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- Ester der Zimtsäure, vorzugsweise d-Methoxyzimtsäure(2-ethylhexyl)ester, 4-Methoxyzimtsäureisopentylester;

- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;

Ganz besonders vorteilhafte wasserlösliche UVB-Filtersubstanzen sind z.B.:

- Salze der 2-Phenylbenzimidazol-5-sulfonsäure wie ihr Natrium-, Kalium- oder ihr Triethanolammonium-Salz, sowie die Sulfonsäure selbst, die durch folgende Struktur gekennzeichnet ist:

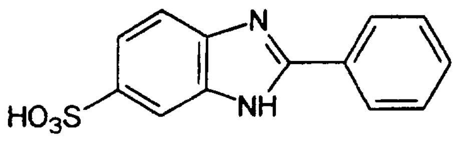

- Sulfonsäure-Derivate von Benzophenonen, vorzugsweise 2-Hydroxy-4-methoxybenzophenon-5-sulfonsäure und deren Salze, beispielsweise das entsprechende Natrium-, Kalium- oder Triethanolammonium-Salz, wobei die 2-Hydroxy-4-methoxybenzophenon-5-sulfonsäure durch folgende Struktur gekennzeichnet ist:

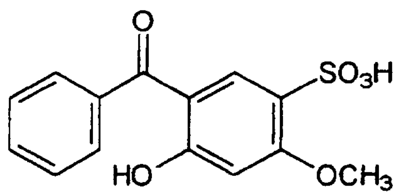

- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und deren Salze, beispielsweise das entsprechende Natrium-, Kalium- oder Triethanolammonium-Salz, wobei die 4-(2-Oxo-3-bornylidenmethyl)benzolsulfonsäure durch folgende Struktur gekennzeichnet ist:

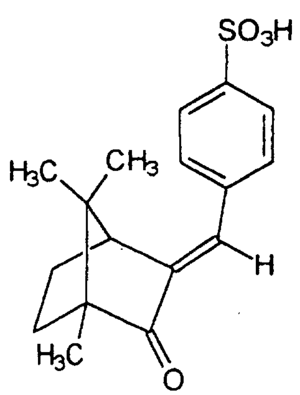

Die 2-Methyl-5-(2-oxo-3-bornylidenmethyl)benzolsulfonsäure ist durch folgende Struktur gekennzeichnet:

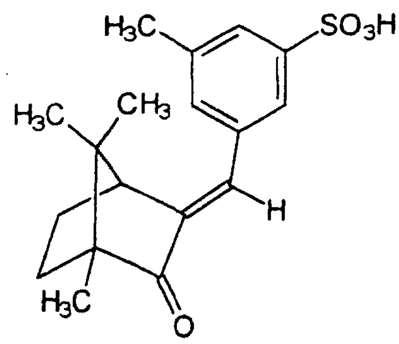

- Das 1,4-di(2-oxo-10-Sulfo-3-bornylidenmethyl-)Benzol und dessen Salze (die entsprechenden 10-Sulfato-verbindungen, beispielsweise das entsprechende Natrium-, Kalium- oder Triethanolammonium-Salz), auch als Benzol-1,4-di(2-oxo-3-bornylidenmethyl-10-Sulfonsäure bezeichnet, wobei die zugrundeliegende Sulfonsäure durch folgende Struktur gekennzeichnet ist:

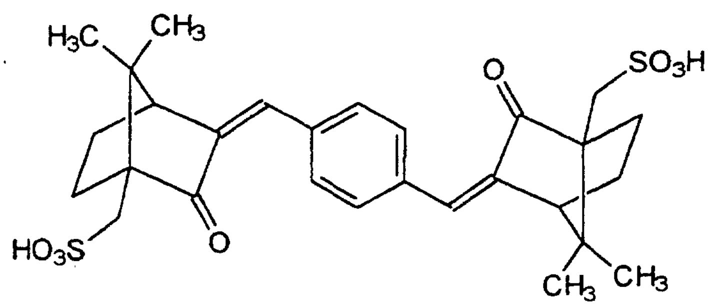

Ganz besonders vorteilhafte Ausführungsformen der vorliegenden Erfindung betreffen daher die Verwendung von einem oder mehreren unsymmetrisch substituierten s-Triazinderivaten in kosmetischen oder dermatologischen Zubereitungen zur Verhinderung

- des durch Einwirkung von Wasser hervorgerufenen Aus- oder Abwaschens der hauteigenen cis- bzw. trans-Urocaninsäure von oder aus der menschlichen Haut oder
- des durch Einwirkung von Wasser hervorgerufenen Aus- oder Abwaschens von künstlich auf die Haut aufgetragener cis- bzw. trans-Urocaninsäure von oder aus der menschlichen Haut,

dadurch gekennzeichnet, daß die Zubereitungen ferner

- eine oder mehrere UV-Filtersubstanzen, die an ihrem Molekülgerüst eine oder mehrere Sulfonsäuregruppen bzw. Sulfonatgruppen tragen,

enthalten.

Die Liste der genannten weiteren UVB-Filter, die in Kombination mit den erfindungsgemäßen Wirkstoffkombinationen verwendet werden können, soll selbstverständlich nicht limitierend sein.

Es kann auch von Vorteil sein, die erfindungsgemäßen Kombinationen mit weiteren UVA-Filtern zu kombinieren, die bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch diese Kombinationen bzw. Zubereitungen, die diese Kombinationen enthalten, sind Gegenstand der Erfindung. Es können die für die UVB-Kombination verwendeten Mengen eingesetzt werden.

Ferner ist vorteilhaft, die erfindungsgemäßen Wirkstoffkombinationen mit weiteren UVA- und/oder UVB-Filtern zu kombinieren.

Vorteilhaft ist insbesondere, Verdicker aus der Gruppe der Polyoxyethylen-Polyoxypropylen-Blockcopolymere zu wählen. Solche Blockcoploymere sind unter der Bezeichnung "Poloxamere" bekannt und zeichnen sich durch

folgende Struktur aus:

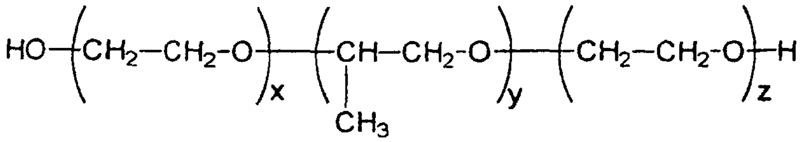

Dabei nimmt x vorteilhaft Werte zwischen 2 und 20 an. y nimmt vorteilhaft Werte zwischen 10 und 50 an. z nimmt vorteilhaft Werte zwischen 2 und 20 an.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

**Beispiel 1**

| | Gew.-% |
|---|---:|
| Methylglucosesesquistearat | 5,00 |
| Capryl-/Caprinsäure-Triglyceride | 10,00 |
| $C_{12-15}$-Alkylbenzoate | 5,00 |
| Dicaprylylether | 5,00 |
| Silikonöl | 1,00 |
| Dioctylbutylamidotriazon | 6,00 |
| Xanthangummi | 0,40 |
| Glycerin | 3,00 |
| Farbstoffe, Konservierungsmittel | q.s. |
| Wasser | ad 100,00 |

**Beispiel 2**

| | Gew.-% |
|---|---:|
| Polyglyceryl(3)-Methylglucosedistearat | 4,00 |
| Sorbitanmonstearat | 2,00 |
| Capryl-/Caprinsäure-Triglyceride | 5,00 |
| $C_{12-15}$-Alkylbenzoate | 5,00 |
| Dicaprylylether | 5,00 |
| Silikonöl | 1,00 |
| Dioctylbutylamidotriazon | 4,00 |
| t-Butylphenyl-methoxyphenylpropandion | 2,00 |
| Octyltriazon | 2,00 |
| 4-Methylbenzylidencampher | 3,00 |
| Natrium-2-Hydroxy-4-methoxybenzophenon-5-sulfonat | 2,00 |
| Natronlauge | 0,30 |
| Xanthangummi | 0,20 |
| Carbomer | 0,20 |
| Glycerin | 5,00 |
| Butylenglykol | 5,00 |
| Glycin | 1,00 |

(fortgesetzt)

| | Gew.-% |
|---|---|
| Farbstoffe, Konservierungsmittel | q.s. |
| Wasser | ad 100,00 |

**Beispiel 3**

| | Gew.-% |
|---|---|
| Polyglyceryl(3)-Methylglucosedistearat | 3,00 |
| Sorbitanmonstearat | 1,00 |
| Capryl-/Caprinsäure-Triglyceride | 5,00 |
| 2-Octyldodecanol | 5,00 |
| Dioctylbutylamidotriazon | 4,00 |
| $TiO_2$ | 2,00 |
| Natronlauge | 0,30 |
| Carbomer | 0,30 |
| Glycerin | 10,00 |
| Farbstoffe, Konservierungsmittel | q.s. |
| Wasser | ad 100,00 |

**Beispiel 4**

| | Gew.-% |
|---|---|
| Methylglucosesesquistearat | 3,00 |
| Polyglyceryl(3)-Methylglucosedistearat | 3,00 |
| $C_{12-15}$-Alkylbenzoate | 8,00 |
| Dicaprylylether | 5,00 |
| Silikonöl | 1,00 |
| Dioctylbutylamidotriazon | 6,00 |
| Urocaninsäure | 1,00 |
| Natronlauge | 0,20 |
| Xanthangummi | 0,10 |
| Carbomer | 0,20 |
| Butylenglykol | 5,00 |
| Glycin | 1,00 |
| Farbstoffe, Konservierungsmittel | q.s. |
| Wasser | ad 100,00 |

**Beispiel 5**

| | Gew.-% |
|---|---|
| Polyglyceryl(3)-Nlethylglucosedistearat | 5,00 |
| Capryl-/Caprinsäure-Triglyceride | 3,00 |
| 2-Octyldodecanol | 3,00 |
| Dicaprylylether | 3,00 |

(fortgesetzt)

| | Gew.-% |
|---|---|
| Silikonöl | 1,00 |
| Dioctylbutylamidotriazon | 4,00 |
| t-Butylphenyl-methoxyphenylpropandion | 1,50 |
| $TiO_2$ | 2, 00 |
| Natrium-2-Hydroxy-4-methoxybenzophenon-5-sulfonat | 2,00 |
| Natronlauge | 0,70 |
| Carbomer | 0,40 |
| Glycerin | 3,00 |
| Glycin | 1,00 |
| Farbstoffe, Konservierungsmittel | q.s. |
| Wasser | ad 100,00 |

**Patentansprüche**

**1.** Kosmetische Verwendung von einem oder mehreren unsymmetrisch substituierten s-Triazinderivaten zur Verhinderung

- des durch Einwirkung von Wasser hervorgerufenen Aus- oder Abwaschens der hauteigenen cis- bzw. trans-Urocaninsäure von oder aus der menschlichen Haut oder
- des durch Einwirkung von Wasser hervorgerufenen Aus- oder Abwaschens von künstlich auf die Haut aufgetragener cis- bzw. trans-Urocaninsäure von oder aus der menschlichen Haut.

**2.** Verwendung von einem oder mehreren unsymmetrisch substituierten s-Triazinderivaten zur Herstellung von dermatologischen Zubereitungen zur Verhinderung

- des durch Einwirkung von Wasser hervorgerufenen Aus- oder Abwaschens der hauteigenen cis- bzw. trans-Urocaninsäure von oder aus der menschlichen Haut oder
- des durch Einwirkung von Wasser hervorgerufenen Aus- oder Abwaschens von künstlich auf die Haut aufgetragener cis- bzw. trans-Urocaninsäure von oder aus der menschlichen Haut.

**3.** Verwendung nach Anspruch 1 oder 2, **dadurch gekennzeichnet, daß** das oder die unsymmetrisch s ubstituierten s-Triazinderivate gewählt w erden a us d er Gruppe d er Substanzen, deren chemische Struktur durch die generische Formel

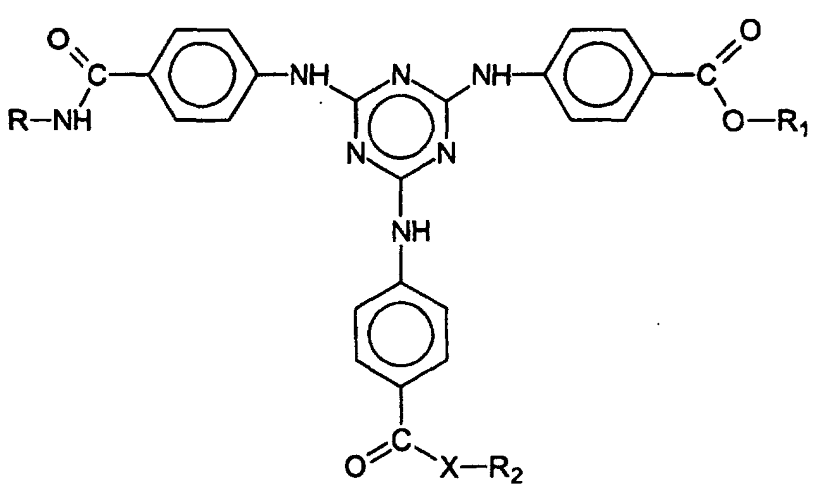

wiedergegeben wird, wobei

R einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, darstellt,

X ein Sauerstoffatom oder eine NH-Gruppe darstellt,

$R_1$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, oder ein Wasserstoffatom, ein Alkalimetallatom, eine Ammoniumgruppe oder eine Gruppe der Formel

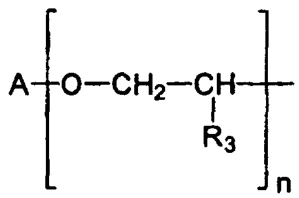

bedeutet, in welcher

A einen verzweigten oder unverzweigten $C_1$-$C_{18}$-Alkylrest, einen $C_5$-$C_{12}$-Cycloalkyl- oder Arylrest darstellt, gegebenenfalls substituiert mit einer oder mehreren $C_1$-$C_4$-Alkylgruppen,

$R_3$ ein Wasserstoffatom oder eine Methylgruppe darstellt,

n eine Zahl von 1 bis 10 darstellt,

$R_2$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, darstellt, wenn X die NH-Gruppe darstellt, und einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, oder ein Wasserstoffatom, ein Alkalimetallatom, eine Ammoniumgruppe oder eine Gruppe der Formel

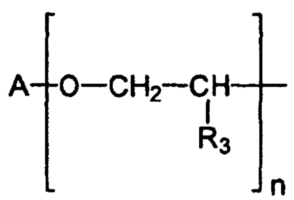

bedeutet, in welcher

A einen verzweigten oder unverzweigten $C_1$-$C_{18}$-Alkylrest, einen $C_5$-$C_{12}$-Cycloalkyl- oder Arylrest darstellt, gegebenenfalls substituiert mit einer oder mehreren $C_1$-$C_4$-Alkylgruppen,

$R_3$ ein Wasserstoffatom oder eine Methylgruppe darstellt,

n eine Zahl von 1 bis 10 darstellt,

wenn X ein Sauerstoffatom darstellt.

4. Verwendung nach Anspruch 1 oder 2, **dadurch gekennzeichnet, daß** als unsymmetrisch substituiertes Triazinderivat das Dioctylbutylamidotriazon gewählt wird.

5. Verwendung nach Anspruch 1 oder 2, **dadurch gekennzeichnet, daß** die die Gesamtmenge an einem oder mehreren unsymmetrisch substituierten s-Triazinderivaten, insbesondere von Dioctylbutylamidotriazon, in den fertigen kosmetischen oder dermatologischen Zubereitungen aus dem Bereich von 0,1 - 15,0 Gew.-%, bevorzugt 0,5 - 8,0 Gew.-% gewählt wird, bezogen auf das Gesamtgewicht der Zubereitungen.

6. Verwendung n ach Anspruch 1 oder 2, **dadurch gekennzeichnet**, d aß d ie kosmetischen oder dermatologischen Zubereitungen Urocaninsäure enthalten, vorzugsweise in Konzentrationen von 0,00001 mg/ml - 60 mg/ml, bezogen auf das Gesamtvolumen der Zubereitungen, besonders bevorzugt von 0,05 mg/ml - 1,0 mg/ml, jeweils bezogen auf das Gesamtvolumen der Zubereitungen.

7. Verwendung nach Anspruch 1 oder 2, **dadurch gekennzeichnet, daß** die kosmetischen Zubereitungen ferner eine oder mehrere UV-Filtersubstanzen, die an ihrem Molekülgerüst eine oder mehrere Sulfonsäuregruppen bzw. Sulfonatgruppen tragen, enthalten.

8. Verwendung nach Anspruch 1 oder 2, **dadurch gekennzeichnet, daß** die kosmetischen Zubereitungen ferner Polyglyceryl(3)-Methylglucosedistearat enthalten.

**Claims**

1. Cosmetic use of one or more unsymmetrically substituted s-triazine derivatives for preventing

- the washing out or washing off, caused by the action of water, of endogenous skin cis- and trans-urocanic acid from human skin or
- the washing out or washing off, caused by the action of water, of cis- and trans-urocanic acid applied artificially to the skin from human skin.

2. Use of one or more unsymmetrically substituted s-triazine derivatives for preparing dermatological preparations for preventing

- the washing out or washing off, caused by the action of water, of endogenous skin cis- and trans-urocanic acid from human skin or
- the washing out or washing off, caused by the action of water, of cis- and trans-urocanic acid applied artificially to the skin from human skin.

3. Use according to Claim 1 or 2, **characterized in that** the unsymmetrically substituted s-triazine derivative(s) is/are chosen from the group of substances whose chemical structure is given by the generic formula

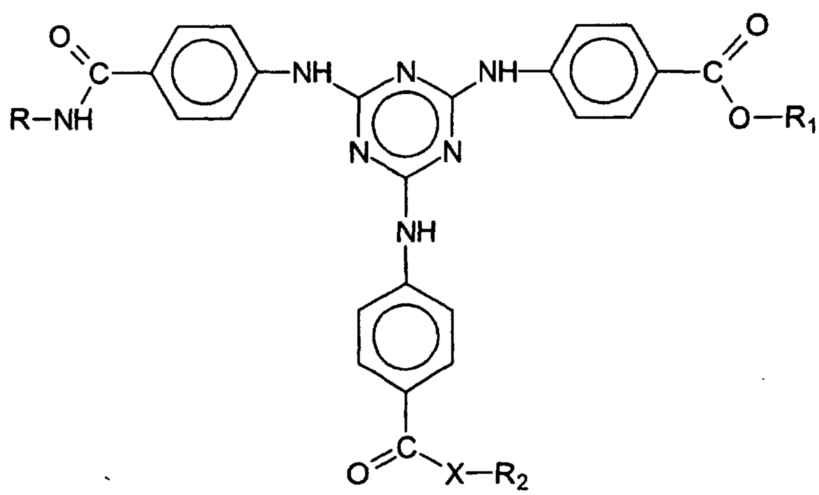

where

R is a branched or unbranched $C_1$-$C_{18}$-alkyl radical, a $C_5$-$C_{12}$-cycloalkyl radical, optionally substituted by one or more $C_1$-$C_4$-alkyl groups,
X is an oxygen atom or an NH group,
$R_1$ is a branched or unbranched $C_1$-$C_{18}$-alkyl radical, a $C_5$-$C_{12}$-cycloalkyl radical, optionally substituted by one or more $C_1$-$C_4$-alkyl groups, or a hydrogen atom, an alkali metal atom, an ammonium group or a group of the formula

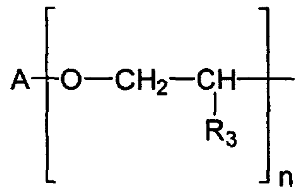

in which

A is a branched or unbranched $C_1$-$C_{18}$-alkyl radical, a $C_5$-$C_{12}$-cycloalkyl or aryl radical, optionally substituted by one or more $C_1$-$C_4$-alkyl groups,
$R_3$ is a hydrogen atom or a methyl group,
n is a number from 1 to 10,

$R_2$ is a branched or unbranched $C_1$-$C_{18}$-alkyl radical, a $C_5$-$C_{12}$-cycloalkyl radical, optionally substituted by one or more $C_1$-$C_4$-alkyl groups, if X is the NH group, and
a branched or unbranched $C_1$-$C_{18}$-alkyl radical, a $C_5$-$C_{12}$-cycloalkyl radical, optionally substituted by one or more $C_1$-$C_4$-alkyl groups, or a hydrogen atom, an alkali metal atom, an ammonium group or a group of the formula

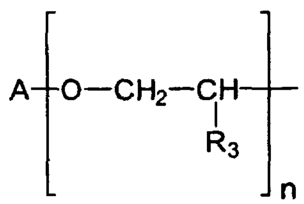

in which

A is a branched or unbranched $C_1$-$C_{18}$-alkyl radical, a $C_5$-$C_{12}$-cycloalkyl or aryl radical, optionally substituted by one or more $C_1$-$C_4$-alkyl groups,
$R_3$ is a hydrogen atom or a methyl group,
n is a number from 1 to 10,
if X is an oxygen atom.

**4.** Use according to Claim 1 or 2, **characterized in that** the unsymmetrically substituted triazine derivative chosen is dioctylbutylamidotriazone.

**5.** Use according to Claim 1 or 2, **characterized in that** the total amount of one or more unsymmetrically substituted s-triazine derivatives, in particular of dioctylbutylamidotriazone, in the finished cosmetic or dermatological preparations is chosen from the range 0.1-15.0% by weight, preferably 0.5-8.0% by weight, based on the total weight of the preparations.

**6.** Use according to Claim 1 or 2, **characterized in that** the cosmetic or dermatological preparations comprise urocanic acid, preferably in concentrations of 0.00001 mg/ml-60 mg/ml, based on the total volume of the preparations, particularly preferably 0.05 mg/ml-1.0 mg/ml, in each case based on the total volume of the preparations.

**7.** Use according to Claim 1 or 2, **characterized in that** the cosmetic preparations further comprise one or more UV filter substances which carry one or more sulphonic acid groups or sulphonate groups on their molecular backbone.

**8.** Use according to Claim 1 or 2, **characterized in that** the cosmetic preparations further comprise polyglyceryl(3) methylglucose distearate.

**Revendications**

1. Utilisation cosmétique d'un ou plusieurs dérivés de triazine-s substitués de façon asymétrique, pour empêcher

   - le détachement ou l'enlèvement par lavage, provoqué par l'action de l'eau, de l'acide cis- ou *trans*-urocanique endogène de la peau, de ou hors de la peau humaine ou
   - le détachement ou l'enlèvement par lavage, provoqué par l'action de l'eau, d'acide *cis*- ou *trans*-urocanique appliqué artificiellement sur la peau, de ou hors de la peau humaine.

2. Utilisation d'un ou plusieurs dérivés de triazine-s substitués de façon asymétrique, pour la production de préparations dermatologiques destinées à empêcher

   - le détachement ou l'enlèvement par lavage, provoqué par action de l'eau, de l'acide *cis*- ou *trans*-urocanique endogène de la peau, de ou hors de la peau humaine ou
   - le détachement ou l'enlèvement par lavage, provoqué par action de l'eau, d'acide *cis*- ou *trans*-urocanique appliqué artificiellement sur la peau, de ou hors de la peau humaine.

3. Utilisation selon la revendication 1 ou 2, **caractérisée en ce que** le ou les dérivés de triazine-s substitués de façon asymétrique sont choisis dans le groupe des substances dont la structure chimique est représentée par la formule générique

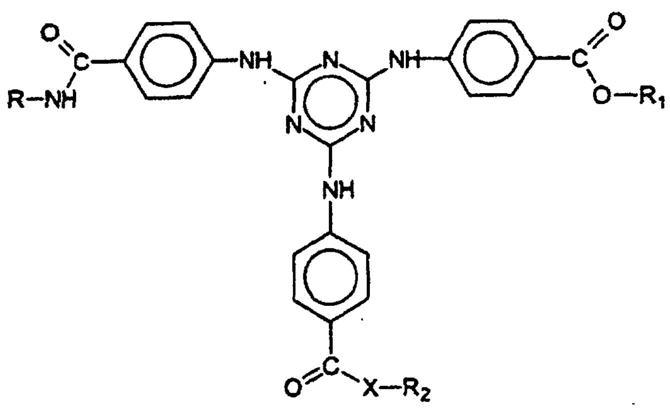

dans laquelle

R représente un radical alkyle en $C_1$-$C_{18}$ ramifié ou non ramifié, un radical cycloalkyle en $C_5$-$C_{12}$, éventuellement substitué par un ou plusieurs groupes alkyle en $C_1$-$C_4$,

X représente un atome d'oxygène ou un groupe NH,

$R_1$ représente un radical alkyle en $C_1$-$C_{18}$ ramifié ou non ramifié, un radical cycloalkyle en $C_5$-$C_{12}$, éventuellement substitué par un ou plusieurs groupes alkyle en $C_1$-$C_4$, ou un atome d'hydrogène, un atome de métal alcalin, un groupe ammonium ou un groupe de formule

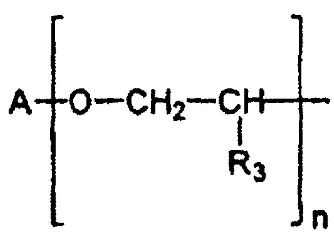

dans laquelle

A représente un radical alkyle en $C_1$-$C_{18}$ ramifié ou non ramifié, un radical cycloalkyle en $C_5$-$C_{12}$ ou

aryle, éventuellement substitué par un ou plusieurs groupes alkyle en $C_1$-$C_4$,

$R_3$ représente un atome d'hydrogène ou un groupe méthyle,

n représente un nombre allant de 1 à 10,

$R_2$ représente un radical alkyle en $C_1$-$C_{18}$ ramifié ou non ramifié, un radical cycloalkyle en $C_5$-$C_{12}$, éventuellement substitué par un ou plusieurs groupes alkyle en $C_1$-$C_4$,
lorsque X représente le groupe NH, et
représente un radical alkyle en $C_1$-$C_{18}$ ramifié ou non ramifié, un radical cycloalkyle en $C_5$-$C_{12}$, éventuellement substitué par un ou plusieurs groupes alkyle en $C_1$-$C_4$ ou un atome d'hydrogène, un atome de métal alcalin, un groupe ammonium ou un groupe de formule

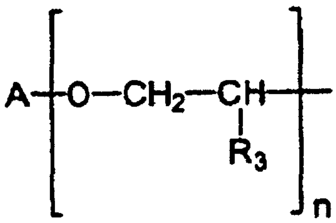

dans laquelle

A représente un radical alkyle en $C_1$-$C_{18}$ ramifié ou non ramifié, un radical cycloalkyle en $C_5$-$C_{12}$ ou aryle, éventuellement substitué par un ou plusieurs groupes alkyle en $C_1$-$C_4$,

$R_3$ représente un atome d'hydrogène ou un groupe méthyle,

n représente un nombre allant de 1 à 10,

lorsque X représente un atome d'oxygène.

4. Utilisation selon la revendication 1 ou 2, **caractérisée en ce que**, en tant que dérivé de triazine substitué de façon asymétrique, on choisit la dioctylbutylamidotriazone.

5. Utilisation selon la revendication 1 ou 2, **caractérisée en ce que** la quantité totale d'un ou plusieurs dérivés de triazine-s substitués de façon asymétrique, en particulier de dioctylbutylamidotriazone, dans les préparations cosmétiques ou dermatologiques finales, est choisie dans la plage allant de 0,1 à 15,0 % en poids, de préférence de 0,5 à 8,0 % en poids, par rapport au poids total des préparations.

6. Utilisation selon la revendication 1 ou 2, **caractérisée en ce que** les préparations cosmétiques ou dermatologiques contiennent de l'acide urocanique, de préférence à des concentrations de 0,00001 mg/ml à 60 mg/ml, par rapport au volume total des préparations, de façon particulièrement préférée de 0,05 mg/ml à 1,0 mg/ml, chaque fois par rapport au volume total des préparations.

7. Utilisation selon la revendication 1 ou 2, **caractérisée en ce que** les préparations cosmétiques contiennent en outre une ou plusieurs substances filtrant les UV, qui portent dans leur squelette moléculaire un ou plusieurs groupes sulfo ou sulfonate.

8. Utilisation selon la revendication 1 ou 2, **caractérisée en ce que** les préparations cosmétiques contiennent en outre du méthylglucosedistéarate de polyglycéryle(3).